# EUROPÄISCHE PATENTSCHRIFT

(11) **EP 1 094 841 B1**
(45) Veröffentlichungstag und Bekanntmachung des Hinweises auf die Patenterteilung: **18.08.2004**
(21) Anmeldenummer: 99931209.3
(22) Anmeldetag: 29.06.1999
(51) Int. Cl.: A61K 9/36, A61K 9/38

(54) **PHARMAZEUTISCHE LEVOTHYROXINZUBEREITUNG**
PHARMACEUTICAL LEVOTHYROXINE PREPARATION
PREPARATION PHARMACEUTIQUE CONTENANT DE LA LEVOTHYROXINE

(30) Priorität: 07.07.1998 DE 19830246
(43) Veröffentlichungstag der Anmeldung: 02.05.2001
(73) Patentinhaber: MERCK PATENT GmbH, 64293 Darmstadt (DE)
(72) Erfinder: SCHREDER, Sven, Alexander, D-69115 Heidelberg (DE); NISCHWITZ, Marion, D-64291 Darmstadt (DE)
(86) Internationale Anmeldenummer: PCT/EP1999/004485
(87) Internationale Veröffentlichungsnummer: WO 2000/002586

(56) Entgegenhaltungen:
- WO-A-97/17951
- US-A- 5 225 204
- US-A- 5 635 209

## Beschreibung

Gegenstand der Erfindung ist eine neue stabile pharmazeutische Zubereitung enthaltend Levothyroxin Natrium, Kaliumiodid, mikrokristalline Cellulose und Bindemittel, die frei von Antioxidantien oder weiteren Hilfsstoffen ist.

Hilfsstoffe sind Stoffe, die eine Bildung von lod verhindern, z.B. Kaliumhydroxid.

Ein mit Thiosulfat als Antioxidans stabilisiertes Thyroxinpräparat ist in der DE 195 41 128 beschrieben.
Ein anderes bekanntes thyroxinhaltiges Handelspräparat Thyreocomb® N (Rote Liste 1998, 74015) enthält den Hilfsstoff Kaliumhydroxid, das die Komproportionierungsreaktion von Iodid und Iodat zu Iod auf die Eduktseite drängt. Auf diese Weise wird eine Iod-Entwicklung unterdrückt.

Ein Präparat enthaltend Levothyroxin Natrium und Kaliumiodid zur Stabilisierung des Wirkstoffs Levothyroxin Natrium ist aus US 5,635,209 bekannt. Die zur Stabilisierung von Levothyroxin Natrium benötigte Menge an Kaliumiodid wird für eine niedrige Dosierung in einem Verhältnis von 4:1 angegeben, z.B. 25 µg an Levothyroxin Natrium und 100 µg Kaliumiodid. Für hohe Dosierungen ist das Verhältnis als 1.5:1 beschrieben, z.B. für 300 µg Levothyroxin Natrium 300-450 µg Kaliumiodid. Zur Stabilisierung von 100 µg Levothyroxin Natrium wurden 300 µg Kaliumiodid benötigt.

Der Wirkstoff Levothyroxin Natrium (= Levothyroxin-Na = LT4) ist gegenüber Licht, Wärme und Sauerstoff empfindlich. Aufgrund dieser bekannten Stabilitätsprobleme liegen pharmazeutische Zubereitungen daher bis zu 20 % überdosiert vor.
Ist neben dem Wirkstoff Levothyroxin-Na in einer pharmazeutischen Zubereitung noch lodid enthalten, so färbt sich diese pharmazeutische Zubereitung bei Lagerung, da das Anion Iodid in Kaliumiodid z.B. zu lod oxidiert werden oder mit Kaliumiodat zu lod komproportionieren kann.

Weiterhin sind die Anforderungen an die in-vitro-Freisetzung für Levothyroxin-Na Tabletten erhöht worden. Der Monographie-Entwurf des Pharmacopeial Forum (Pharm. Preview, **1995**, *21*, 1459-1461) sieht vor, neben dem gültigen Test 1 (Phosphatpuffer pH 7,4, in 80 Minuten > 55 %) den Test 2 (Wasser in 45 Minuten > 70%) zuzulassen.

Der Erfindung lag die Aufgabe zugrunde, neue Arzneimittel in Form von pharmazeutischen Zubereitungen zur Verfügung zu stellen, die bessere Eigenschaften besitzen als bekannte, für die gleichen Zwecke verwendbare Arzneimittel.

Diese Aufgabe wurde durch das Auffinden der neuen Zubereitung gelöst.

Die erfindungsgemäße neue Zubereitung zeigt im wesentlichen keine Verfärbung und hat eine verbesserte Stabilität. Sie kann als Thyroidhormon-Kombinationspräparat, bedingt durch den hohen Anteil an Iodid als zweiten Wirkstoff, bei euthyreotem lodmangelstruma und/oder bei Rezidivprophylaxe nach Resektion einer lodmangelstruma verwendet werden.
Der Wirkstoff Iodid kann als Anion nur in Gegenwart eines stabilisierenden Kations, z.B. Kalium (+), und damit als Salz in einer pharmazeutischen Zubereitung enthalten sein. 130 µg Kaliumiodid entsprechen 100 µg Iodid.

Eine Verfärbung der erfindungsgemäßen Zubereitung wird vermieden, da eine Bildung von freiem lod verhindert wird.

Diese neue Zubereitung hat weiterhin eine sehr gute Wirkstoff-Freisetzung in vitro.

Gegenstand der Erfindung ist vorzugsweise eine pharmazeutische Zubereitung wie beschrieben, dadurch gekennzeichnet, daß sie 5 bis 400 µg, vorzugsweise 10 bis 300 µg, insbesondere 50 bis 200 µg an Levothyroxin Natrium und 5 bis 400 µg, vorzugsweise 10 bis 300 µg, insbesondere 25 bis 200 µg Kaliumiodid enthält.

Gegenstand der Erfindung ist ferner vorzugsweise eine pharmazeutische Zubereitung wie beschrieben, dadurch gekennzeichnet, daß sie Levothyroxin Natrium mikronisiert mit einer Korngröße zwischen 5 und 25 µm (zu 95%), besonders bevorzugt mit einer Korngröße zwischen 5 und 15 µm (zu 95%) enthält.

Gegenstand der Erfindung ist weiterhin vorzugsweise eine pharmazeutische Zubereitung wie beschrieben, dadurch gekennzeichnet, daß als Bindemittel Hydroxypropylmethylcellulose und/oder Gelatine enthalten ist.

Besonders bevorzugt ist eine pharmazeutische Zubereitung wie beschrieben, dadurch gekennzeichnet, daß es sich um eine feste Zubereitung in Form von Tabletten handelt.

Besonders bevorzugte Ausführungsformen enthalten 50, 75 oder 100 µg an Levothyroxin Natrium und jeweils 100 µg an Iodid, wobei 100 µg Iodid der Menge von 130 µg Kaliumiodid entspricht. Eine ganz besonders bevorzugte Ausführungsform enthält 100 µg Levothyroxin Natrium und 100 µg Iodid.
Aufgrund der bekannten Instabilität des Levothyroxin-Na wird in den Formulierungen dieser Wirkstoff zu 5 % überdosiert.

Die erfindungsgemäße Zubereitung weist eine überraschende Stabilität auf, wenn als Bindemittel Hydroxypropylmethylcellulose und/oder Gelatine verwendet wird. Gleichzeitig wird überraschenderweise eine Bildung von Iod unterdrückt, ohne daß eine Zumischung von Antioxidantien oder weiteren Hilfsstoffen nötig wird.

Die Daten der Stabilitätsuntersuchungen sind in den Tabellen I und II am Beispiel der Chargen 005204 (13/97) und 004609 (3/96) angegeben. Basierend auf den Ergebnissen zeigt sich, daß die erfindungsgemäßen Tabletten, die Levothyroxin Natrium (100 µg) und Iodid (100 µg) enthalten, mindestens 2 Jahre stabil sind, wenn sie bei Temperaturen unter 30° C gelagert werden. In diesem Zeitraum wird ebenfalls keine Braunfärbung der pharmazeutischen Zubereitung beobachtet, d.h. keine Bildung von lod.

Ferner wird die Freisetzung des Wirkstoffs Levothyroxin Natrium begünstigt, wenn der Wirkstoff in mikronisierter Form eingesetzt wird. Levothyroxin Natrium ist üblicherweise sowohl in Wasser als auch in Ethanol sehr schwer löslich. Mit einer Partikelgröße zwischen 5 und 25 µm (zu 95%), besonders bevorzugt zwischen 5 und 15 µm, erfolgt jedoch eine Freisetzung des Wirkstoffs, die beiden Testsystemen entspricht (Tabellen I und II).

**Tabelle I**

| Stabilität und Freisetzung der Charge 005204 (13/97); Levothyroxin-Na (LT4) 100 µg/lodid 100 µg Tabletten; hergestellt analog Beispiel 1: PP-Röhre 25°/60% | | | | | | | |
|---|---|---|---|---|---|---|---|
| Dauer Datum (Auslagerung) Datum (Untersuchung) | Gehalt LT4 [µg] | Gehalt Iodid [µg] | Freisetzung mit Puffer [%] | Freisetzung mit Wasser [%] | Wassergehalt nach KF [%] | Zerfalls zeit [sec] | Bruchfestigkeit [N] |
| Startwert | 108,3 | 99,5 | 30min: 94,3 | 15min: 91,3 | 2,51 | 43-55 | 42-50 |
| | | | 60min: 99,2 | 30min: 93,5 | | | |
| | | | 80min: 100,6 | 45min: 95,1 | | | |
| 13 Wochen | 106,9 | 104,2 | | 15min: 85,9 | 3,28 | 50-60 | 48-55 |
| 18.07.1997 | | | | 30min: 89,9 | | | |
| 13.02.1998 | | | | 45min: 91,2 | | | |
| 26 Wochen | 102,1 | 102,8 | | 15min: 89,5 | 3,14 | 50-68 | 41-48 |
| 17.10.1997 | | | | 30min: 91,6 | | | |
| 21.02.1998 | | | | 45min: 95,1 | | | |
| 39 Wochen | 100,6 | 103,2 | | 15min: 84,6 | 3,28 | 49-60 | 41-49 |
| 19.01.1998 | | | | 30min: 88,1 45min: 89,3 | | | |
| 52 Wochen | 100,9 | 103,2 | | 15min: 84,6 | 3,50 | 40-55 | 41-49 |
| | | | | 30min: 87,2 | | | |
| | | | | 45min: 89,5 | | | |
| Bemerkung: 5% Überdosierung von LT4 | | | | | | | |

**Tabelle II**

| Stabilität und Freisetzung der Charge 004609 (3/96); Levothyroxin-Na (LT4) 100 µg/lodid 100 µg Tabletten; hergestellt analog Beispiel 1: PP-Blister 25°/60% | | | | | | | |
|---|---|---|---|---|---|---|---|
| Dauer Datum (Auslagerung) Datum (Untersuchung) | Gehalt LT4 [µg] | Gehalt Iodid [µg] | Freisetzung mit Puffer [%] | Freisetzung mit Wasser [%] | Wassergehalt nach KF [%] | Zerfallszeit [sec] | Bruchfestigkeit [N] |
| Startwert | 108,3 | 103,6 | 30min: 105,3 | 15min: 101,0 | 3,18 | | |
| | | | 60min: 105,3 | 30min: 102,9 | | | |
| | | | 80min: 103,1 | 45min: 106,1 | | | |
| 13 Wochen | 104,9 | 100,2 | 15min: 94,6 | | 4,8 | | |
| 23.12.1996 | | | 30min: 96,3 | | | | |
| | | | 45min: 96,7 | | | | |
| 26 Wochen | 104,8 | 103,6 | 15min: 96,1 | | 5,34 | | |
| 10.04.1997 | | | 30min: 97,2 | | | | |
| 24.02.1998 | | | 45min: 97,4 | | | | |
| 52 Wochen | 101,5 | 102,7 | | 15min: 94,1 | 6,26 | 37-50 | 39-44 |
| 29.09.1997 | | | | 30min: 95,3 | | | |
| | | | | 45min: 95,8 | | | |
| 78 Wochen | 99,72 | 104,6 | | 15min: 95,8 | 5,69 | 30-38 | 35-40 |
| 24.02.1998 | | | | 30min: 97,5 | | | |
| | | | | 45min: 98,6 | | | |
| Bemerkung: 5% Überdosierung von LT4 | | | | | | | |

Die analytischen Daten werden nach üblichen und bekannten Methoden bestimmt.

Gegenstand der Erfindung ist auch ein Verfahren zur Herstellung einer pharmazeutischen Zubereitung enthaltend Levothyroxin Natrium und Kaliumiodid, dadurch gekennzeichnet, daß man in einer Wirbelschichtgranulation Levothyroxin Natrium und Kaliumiodid, die in suspendierter Form in wäßriger Hydroxypropylmethylcellulose- und/oder Gelatinelösung vorliegen, auf die mikrokristalline Cellulose aufsprüht, anschließend ein Spreng- und Schmiermittel zumischt und die Mischung zu Tabletten verpresst.

Die Lösung von Hydroxypropylmethylcellulose und Kaliumiodid in Wasser und die Suspension von Levothyroxin Natrium in Wasser erfolgt bei Temperaturen zwischen 5 und 40°C, bevorzugt zwischen 10 und 35°C, besonders bevorzugt zwischen 15 und 30°C.
Die Temperatur während der Granulation liegt zwischen 60 und 80°C, bevorzugt zwischen 65 und 75°C am Inlet und zwischen 10 und 50°C, bevorzugt zwischen 20 und 40°C am Outlet. Der Sprühdruck liegt bei dem erfindungsgemäßen Verfahren zwischen 3 und 5 bar.

Gegenstand der Erfindung ist weiter ein Verfahren wie beschrieben, dadurch gekennzeichnet, daß als Sprengmittel Croscarmellose Natrium und als Schmiermittel Magnesiumstearat verwendet wird.

Weitere Träger- oder Zusatzstoffe können zugesetzt werden, wie z.B. Bindemittel, Farbstoffe, Gleitmittel, Süßungsmittel und/oder Aromastoffe.

Als Gleit- oder Schmiermittel sind bevorzugt z.B. Talk, Stärke, Magnesiumund Calciumstearat, Borsäure, Paraffin, Kakaobutter, Macrogol, Leucin oder Natriumbenzoat, ganz besonders bevorzugt ist Magnesiumstearat.

Die erfindungsgemäße Zubereitung kann ohne die Verwendung organischer Lösungsmittel hergestellt werden.

Die nachfolgenden Beispiele betreffen die Herstellung und die Zusammensetzung der erfindungsgemäßen pharmazeutischen Zubereitung:

### Beispiel 1

Die folgenden Mengen werden benötigt, um beispielsweise 50.000 Tabletten herzustellen:

| Levothyroxin 100 µg/ lodid 100 µg | |
|---|---|
| Inhaltsstoff | Menge [g] |
| | |
| Levothyroxin Natrium* | 5,25 |
| Kaliumiodid | 6,54 |
| Hydroxypropylmethylcellulose | 175,00 |
| mikrokristalline Cellulose | 4125,70 |
| Croscarmellose Natrium | 175,00 |
| Magnesiumstearat | 12,50 |
| Wasser, gereinigt** | 3259,00 |

| | |
|---|---|
| * Eine 5 %ige Überdosierung für Levothyroxin Natrium wurde mit eingerechnet. | |
| ** Das Wasser wird durch Trocknen wieder entfernt. | |

### Herstellung:

1. Hydroxypropylmethylcellulose und Kaliumiodid werden unter Rühren in ca. 90 % des Wassers bei Raumtemperatur gelöst.
   Levothyroxin Natrium wird in ca. 10 % des Wassers bei Raumtemperatur suspendiert.
   Die Suspension wird danach unter Zuhilfenahme eines Mischers mit der Hydroxypropylmethylcellulose-Kaliumiodid-Lösung vereinigt.
2. Die mikrokristalline Cellulose wird in einem Wirbelschichtgranulator vorgelegt. Die Granulierflüssigkeit wird über das Pulver gesprüht. Während der Granulation wird die Temperatur am Inlet bei ungefähr 70° C (± 5°C), am Outlet zwischen 20 und 40° C gehalten. Der Sprühdruck liegt zwischen 3 und 5 bar.
   Nach dem Sprühen wird das Granulat getrocknet bis am Outlet eine Temperatur von ungefähr 40° C erreicht ist.

Anschließend wird das trockene Granulat nach bekannten Methoden gesiebt (1 mm) (=Mischung a).

Croscarmellose Natrium und Magnesiumstearat werden entsprechend gesiebt. Anschließend werden die Komponenten zusammen mit Mischung a in einem Trommelmixer 10 Minuten miteinander vermischt.

Die pressfertige Mischung wird danach zu Tabletten verpreßt.

### Beispiel 2

Zusammensetzung einer 90 mg (± 3 mg) Tablette, die 105 µg Levothyroxin Natrium und 130 µg Kaliumiodid und somit 100 µg Iodid enthält:

| | |
|---|---|
| Levothyroxin Natrium | 0.105 mg |
| Kaliumiodid | 0.1308 mg |
| Wasser* | 65.00 mg |
| Hydroxypropylmethylcellulose | 3.50 mg |
| Cellulose, mikrokristallin | 82.514 mg |
| Croscarmellose Natrium | 3.50 mg |
| Magnesiumstearat | 0.25 mg |
| | 90.00 mg |
| Levothyroxin Natrium ist um 5 % überdosiert. | |

| | |
|---|---|
| * Wasser wird durch die Trocknung entfernt. | |

### Vergleichsbeispiel

Die folgenden Mengen werden benötigt, um beispielsweise 60.000 Tabletten herzustellen:

| Levothyroxin 100 µg/ KI 300 µg | |
|---|---|
| Inhaltsstoff | Menge [g] |
| | |
| Levothyroxin Natrium* | 7,03 |
| Kaliumiodid | 17,99 |
| Cellulose | 5885,72 |
| Croscarmellose Natrium | 50,00 |
| Magnesiumstearat | 25,00 |
| Wasser, gereinigt** | 4140,00 |

| | |
|---|---|
| * Eine 5 %ige Überdosierung für Levothyroxin Natrium wurde mit eingerechnet. | |
| ** Das Wasser wird durch Trocknen wieder entfernt. | |

### Herstellung:

1. Levothyroxin Natrium (1,05%) und ca. 10% der Cellulose werden gesiebt und 20 Minuten in einem Turbulamischer gemischt.
2. Das Kaliumiodid (18%) wird in 60% des Wassers gelöst. Mit dieser Lösung werden 54% der Cellulose befeuchtet und die behandelte Cellulose geknetet und gesiebt (1 mm). Nach dem Trocknen im Vakuum bei Raumtemperatur wird das entstandene Granulat (0,1% an KI) durch ein 0,75 mm Sieb gesiebt.
3. Die verbliebene Menge Kaliumiodid (82%) wird in der dritten Stufe analog zu Stufe 2 auf Cellulose aufgebracht. Man erhält ein gesiebtes Granulat mit 0,7% an KI.
4. Natriumcarboxymethylcellulose (Croscarmellose Natrium) und Magnesiumstearat werden entsprechend gesiebt (0,5 mm).
5. Die aus den Stufen 1 bis 4 erhaltenen Granulate oder Feststoffgemische werden vereinigt und 20 Minuten nach bekannten Methoden gemischt. Das Tablettieren erfolgt auf einer Rundläuferpresse (13 kN Preßkraft).

Die Tabletten sind gelb bis braun gefärbt.

## Patentansprüche

1. Pharmazeutische Zubereitung enthaltend Levothyroxin Natrium, Kaliumiodid, mikrokristalline Cellulose und Hydroxypropylmethylcellulose und/oder Gelatine.

2. Pharmazeutische Zubereitung nach Anspruch 1, **dadurch gekennzeichnet, daß** 5 bis 400 µg an Levothyroxin Natrium und 5 bis 400 µg Kaliumiodid enthalten sind.

3. Pharmazeutische Zubereitung nach Anspruch 1, **dadurch gekennzeichnet, daß** Levothyroxin Natrium mikronisiert mit einer Korngröße zwischen 5 und 25 µm enthalten ist.

4. Pharmazeutische Zubereitung nach einem oder mehreren der Ansprüche 1 bis 3, **dadurch gekennzeichnet, daß** es sich um eine feste Zubereitung in Form von Tabletten handelt.

5. Verfahren zur Herstellung einer pharmazeutischen Zubereitung nach Anspruch 4, **dadurch gekennzeichnet, daß** man in einer Wirbeeschichtgranulation Levothyroxin Natrium und Kaliumiodid, die in suspendierter Form in wäßriger Hydroxypropylmethylcellulose- und/oder Gelatinelösung vorliegen, auf die mikrokristalline Cellulose aufsprüht, anschließend ein Spreng- und Schmiermittel zumischt und die Mischung zu Tabletten verpresst.

6. Verfahren nach Anspruch 5, **dadurch gekennzeichnet, daß** als Sprengmittel Croscarmellose Natrium und als Schmiermittel Magnesiumstearat verwendet wird.

## Claims

1. Pharmaceutical preparation comprising levothyroxine sodium, potassium iodide, microcrystalline cellulose and hydroxypropylmethylcellulose and/or gelatine.

2. Pharmaceutical preparation according to Claim 1, **characterised in that** it comprises from 5 to 400 µg of levothyroxine sodium and from 5 to 400 µg of potassium iodide.

3. Pharmaceutical preparation according to Claim 1, **characterised in that** it comprises micronised levothyroxine sodium having a particle size of between 5 and 25 µm.

4. Pharmaceutical preparation according to one or more of Claims 1 to 3, **characterised in that** it is a solid preparation in the form of tablets.

5. Process for the preparation of a pharmaceutical preparation according to Claim 4, **characterised in that** levothyroxine sodium and potassium iodide in suspended form in aqueous hydroxypropylmethylcellulose and/or gelatine solution are sprayed onto the microcrystalline cellulose in a fluidised-bed granulation, a disintegrant and lubricant are subsequently admixed, and the mixture is pressed to form tablets.

6. Process according to Claim 5, **characterised in that** the disintegrant used is croscarmellose sodium and the lubricant used is magnesium stearate.

## Revendications

1. Préparation pharmaceutique comprenant du sodium lévothyroxine, du iodure de potassium, de la cellulose microcrystalline et de l'hydroxypropylméthylcellulose et/ou de la gélatine.

2. Préparation pharmaceutique selon la revendication 1, **caractérisée en ce qu'**elle comprend de 5 à 400 µg de sodium lévothyroxine et de 5 à 400 µg de iodure de potassium.

3. Préparation pharmaceutique selon la revendication 1, **caractérisée en ce qu'**elle comprend du sodium lévothyroxine micronisé qui présente une dimension de particule entre 5 et 25 µm.

4. Préparation pharmaceutique selon une ou plusieurs des revendications 1 à 3, **caractérisée en ce qu'**il s'agit d'une préparation solide sous forme de comprimés.

5. Procédé pour la préparation d'une préparation pharmaceutique selon la revendication 4, **caractérisé en ce que** le sodium lévothyroxine et le iodure de potassium sous forme suspendue dans une solution aqueuse d'hydroxypropylméthylcellulose et/ou de gélatine sont pulvérisés sur la cellulose microcrystalline selon une granulation en lit fluidisé, un agent délitant et un lubrifiant sont ensuite ajoutés au mélange et le mélange est pressé pour former des comprimés.

6. Procédé selon la revendication 5, **caractérisé en ce que** l'agent délitant utilisé est du croscarmellose sodique et le lubrifiant utilisé est du stéarate de magnésium.
